# EUROPEAN PATENT APPLICATION

(11) **EP 0 967 204 A1**
(43) Date of publication of application: **29.12.1999**
(21) Application number: 98900692.9
(22) Date of filing: 22.01.1998
(51) Int. Cl.: C07D 209/44

(54) **PROCESS FOR PRODUCING BENZYLSUCCINIC ACID DERIVATIVES**

(30) Priority: 29.01.1997 JP 5083997
(71) Applicant: Kissei Pharmaceutical Co. Ltd., Matsumoto-shi, Nagano 399 (JP)
(72) Inventor: KAMIJO, Tetsuhide, Shiojiri-shi,Nagano 399-07 (JP); YAMAGUCHI, Toshiaki, Matsumoto-shi,Nagano 390 (JP); YANAGI, Takashi, Minamiazumi-gun,Nagano 399-83 (JP)
(74) Representative: Baverstock, Michael George Douglas
(86) International application number: JP9800231
(87) International publication number: WO9832736

(57) **Abstract**

The present invention relates to a novel production process which comprises allowing a benzylsuccinic acid derivative represented by the formula: 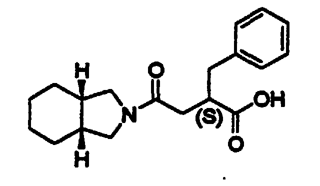 to react with a benzyl halide such as benzyl bromide to give a benzyl ester derivative represented by the formula: 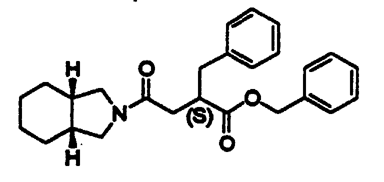 , then purifying and debenzylating the same, optionally followed by the conversion into a salt thereof to thereby efficiently and stably supply highly pure benzylsuccinic acid derivatives useful as a remedy for diabetes.

## Description

### Technical Field

The present invention relates to a process for producing a benzylsuccinic acid derivative represented by the formula: 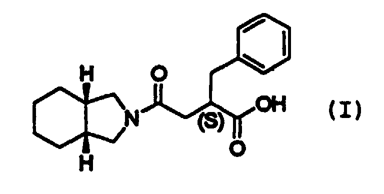 (wherein the carbon atom marked with (S) represents a carbon atom in S configuration) and salts thereof, which have excellent hypoglycemic effects and are useful as a remedy for diabetes.

More particularly, the present invention relates to a process for producing the highly pure benzylsuccinic acid derivative represented by the above formula (I), which comprises allowing the benzylsuccinic acid derivative represented by the formula: 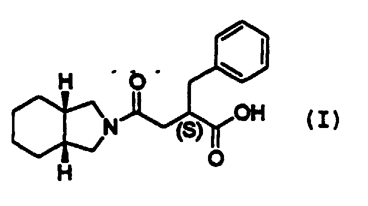 (wherein the carbon atom marked with (S) has the same meaning as defined above) and salts thereof containing byproducts to react with a compound represented by the general formula: 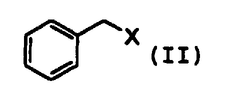 (wherein X represents a hydroxy group, a chlorine atom or a bromine atom) to prepare a benzyl ester derivative represented by the formula: 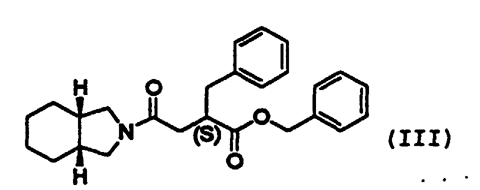 (wherein the carbon atom marked with (S) has the same meaning as defined above), purifying and debenzylating the resulting benzyl ester derivative, and converting the resulting benzylsuccinic acid derivative into a salt thereof as occasion demands, and to the benzylsuccinic acid derivative represented by the above formula (I) and salts thereof which are obtained by the above production process.

### Background Art

Some processes for producing the benzylsuccinic acid derivative represented by the above formula (I) and salts thereof, which are useful as a remedy for diabetes, have already been proposed (Japanese Patent Application Publication (kokai) No. Hei 4-356459, ibid. Hei 6-340622, ibid. Hei 6-340623).

For example, a production process which comprises allowing a benzylsuccinic acid derivative represented by the formula: 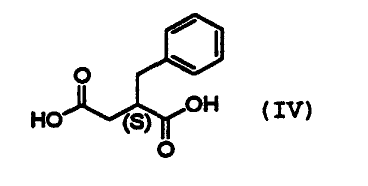 (wherein the carbon atom marked with (S) has the same meaning as defined above) or a reactive functional derivative thereof to react with imidazole, carbonyldiimidazole, oxalyldiimidazole, thionyldiimidazole or thiocarbonyldiimidazole to give an imidazolyl derivative represented by the formula: 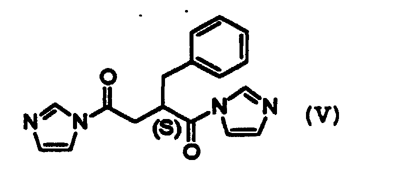 (wherein the carbon atom marked with (S) has the same meaning as defined above), allowing the resulting compound to react with a cyclic amine represented by the formula: 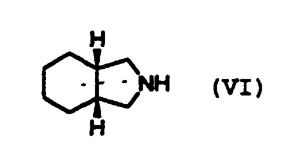 and hydrolyzing the resulting compound, has been disclosed.

Since the benzylsuccinic acid derivative represented by the above formula (I) obtained by the above production processes has a low melting point and is difficult to purify itself, purification after converting it into a calcium salt and the like has been tried.

However, when purification is conducted by recrystallization using these salts, the efficiency of purification was poor, and therefore repeated recrystallization procedure is necessary to ensure quality as a drug. As a result, the yield declines and there is a problem that the benzylsuccinic acid derivative represented by the above formula (I) purified in this manner has an unevenness in purity between lots.

It is considered that the benzylsuccinic acid derivative (I) prepared by the present invention will be administered long term because it is a remedy for diabetes. Therefore, development of a novel production process for efficient and stable supply of the highly pure benzylsuccinic acid derivative is greatly desired.

The present inventors have studied earnestly to solve the above problem. As a result, it was found that the benzyl ester derivative represented by the above formula (III) has a very suitable crystalline property for the purification procedure by recrystallization. That is, it was found that in the step for producing the benzylsuccinic acid derivative represented by the above formula (I), byproducts in the reaction mixture can be efficiently removed by converting it into the benzyl ester derivative represented by the above formula (III) temporarily and purifying by recrystallization, and therefore the highly pure benzylsuccinic acid derivative represented by the above formula (I) can be prepared stably thereby forming the basis of the present invention.

### Disclosure of the Invention

The present invention relates to a process for producing the benzylsuccinic acid derivative represented by the formula: 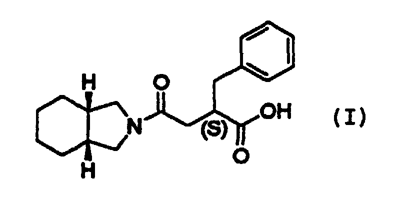 (wherein the carbon atom marked with (S) represents a carbon atom in *S* configuration) and salts thereof which comprises debenzylating the benzyl ester derivative represented by the formula: 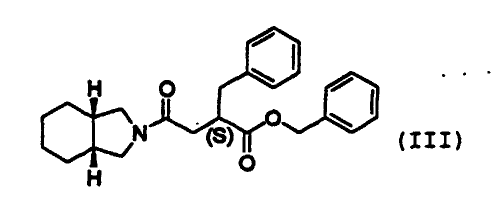 (wherein the carbon atom marked with (S) represents a carbon atom in *S* configuration) and converting the resulting benzylsuccinic acid derivative into a salt thereof as occasion demands.

The present invention relates to a use of the benzyl ester derivative represented by the above formula (III) in the production of the benzylsuccinic acid represented by the above formula (I) and salts thereof.

The present invention relates to the benzylsuccinic acid derivative represented by the above formula (I) produced by debenzylating the benzyl ester derivative represented by the above formula (III) and converting into a salt thereof as occasion demands.

Accordingly, the present invention is to prepare the benzylsuccinic acid derivative represented by the above formula (I) and salts thereof by allowing the benzylsuccinic acid derivative represented by the above formula (I) to react with benzyl chloride or benzyl bromide in the presence of a base in an inert organic solvent or to react with benzyl alcohol in the presence of a condensing agent to prepare a benzyl ester derivative represented by the formula (III) temporarily, purifying the same by recrystallization to remove byproducts in the reaction mixture, debenzylating the resulting derivative in the usual way and converting into a salt thereof as occasion demands. The highly pure benzylsuccinic acid derivative represented by the above formula (I) and salts thereof can be prepared efficiently and reproducibly by the above mentioned method.

As an inert organic solvent used in the process for producing the benzyl ester derivative represented by the above formula (III) of the present invention, organic solvents such as ethyl acetate, toluene, acetone, N,N-dimethylformamide, dimethylsulfoxide, acetonitrile, methylene chloride, chloroform, tetrahydrofuran and benzene can be exemplified. As a base used in the process of the present invention, inorganic bases such as potassium carbonate and organic tertiary amines such as 1,8-diazabicyclo[5.4.0]-7-undecene, triethylamine, N-methylmorpholine and diisopropylethylamine can be exemplified. As a condensing agent used in the process of the present invention, halogenating agents such as thionyl chloride, oxalyl chloride, phosphorus trichloride, phosphorus pentachloride and phosphorus oxychloride, and condensing agents used for general peptide synthesis such as N,N'-dicyclohexylcarbodiimide, N,N'-carbonyldiimidazole, N,N'-disuccinimidylcarbonate and 1-ethyl-3-(3-dimethylaminopropyl) carbodiimide hydrochloride can be exemprified. As a recrystallization solvent used in the process of the present invention, organic solvents such as ethyl acetate and organic mixed solvents such as ethyl acetate-hexane can be exemplified.

As a method for debenzylating the benzyl ester represented by the above formula (III), hydrolysis using an alkaline metal hydroxide such as sodium hydroxide or potassium hydroxide, and catalytic reduction using a metal catalyst such as palladium in an organic solvent such as alcohol can be exemplified.

As a salt of the benzylsuccinic acid derivative represented by the above formula (I), pharmaceutically acceptable salts such as a calcium salt, a sodium salt, a potassium salt and an L-arginine salt can be illustrated. These salts can be readily prepared from the benzylsuccinic acid represented by the above formula (I) in the usual way.

To addition, the benzylsuccinic acid derivative represented by the above formula (I) of the present invention and salts thereof may form their solvates with pharmaceutically acceptable solvents such as water and ethanol.

A preferred embodiment of the present invention is as follows.

The benzylsuccinic acid derivative represented by the above formula (I) is dissolved in ethyl acetate, about 2 mole equivalent of potassium carbonate is added to the solution, about 1.2 mole equivalent of benzyl bromide or benzyl chloride is added to the mixture with stirring, and the resulting mixture is heated with stirring. After allowing to stand to cool, ice-water is added to the reaction mixture and the organic layer is separated. The organic layer is washed with brine and dried over anhydrous magnesium sulfate, and the solvent is removed under reduced pressure. The resulting residue is crystallized from ethyl acetate-hexane and then recrystallized from ethyl acetate. After the resulting benzyl ester derivative represented by the above formula (III) is alkali hydrolyzed, the resulting compound is converted into a salt as occasion demands to give the benzylsuccinic acid derivative represented by the above formula (I) and salts thereof.

Thus, the benzyl ester derivative represented by the above formula (III) is very suitable for purification procedure, in crystaline property, solubility and the like. Therefore, the production process of the present invention is an excellent method enabling stable supply of the highly pure benzylsuccinic acid derivative represented by the above formula (I) and salts thereof in industrial production.

### Best Mode for Carrying Out the Invention

The present invention is further illustrated in more detail by way of the following Reference Example, Examples and Comparative Example. However, the present invention is not limited thereto. All melting points of compounds in Examples are uncorrected. The chemical purity of (*S*)-2-benzyl-3-(cis-hexahydro-2-isoindolinylcarbonyl)propionic acid and calcium bis[(*S*)-2-benzyl-3-(cis-hexahydro-2-isoindolinylcarbonyl)propionate] dihydrate was analyzed by high performance liquid chromatography under the following conditions using a solution of each sample (50 mg) in methanol (10 mL) as a sample solution.
Column: WAKOSIL 5C18-200T (4.6 ⌀ x 150mm)
Eluent: 0.04M aqueous (NH₄)₂HPO₄ solution/ CH₃CN = 3/1 (V/V)
Flow rate: 1.0mL/min
Wavelength for detection: 259nm
Column temperature: 0°C

### Reference Example 1

### (S)-2-Benzyl-3-(cis-hexahydro-2-isoindolinylcarbonyl)propionic acid

Triethylamine 42.5 g was dissolved in ethyl acetate 250mL, and imidazole 14.3 g and (*S*)-benzylsuccinic acid 20.8 g were added to the solution with stirring. Thionyl chloride 25.0 g was added to the solution at -15°C with stirring, and the mixture was allowed to react for 4 hours at -15 to 0°C. cis-Hexahydroisoindoline 12.5 g was added to the reaction mixture, and the mixture was allowed to react overnight at 0°C to room temperature. To the reaction mixture was added 1N hydrochloric acid 200 mL, and the organic layer was separated, washed with water and extracted with 1N aqueous sodium hydroxide solution. The aqueous layer was washed with ethyl acetate, neutralized with 1N hydrochloric acid and extracted with ethyl acetate. The organic layer was washed with brine and dried over anhydrous magnesium sulfate. The solvent was removed under reduced pressure to give (S)-2-benzyl-3-(cis-hexahydro-2-isoindonylcarbonyl)propionic acid 30.6 g as a colorless viscous oil. Chemical purity: 93.2%
¹H-NMR (CDCl₃, 270MHz) δ ppm:
1.15-1.7 (8H, m), 2.05-2.3 (2H, m), 2.35-2.55 (2H, m), 2.65-3.5 (7H, m),7.1-7.4 (5H, m)
IR(neat): 1735, 1605 cm⁻¹

### Example 1

### Benzyl (S)-2-benzyl-3- cis-hexahydro-2-isoindonylcarbonyl)-propionate

(S)-2-benzyl-3-(cis-hexahydro-2-isoindonylcarbonyl)-propionic acid 27.54 g was dissolved in ethyl acetate 200 mL. To the solution was added potassium carbonate 24.9g, and benzyl bromide 12.8 mL was added to the mixture at room temperature with stirring. The resulting mixture was allowing to react for 6 hours at 80°C. After allowing to stand to cool, ice-water 200 mL was added to the reaction mixture, and the organic layer was separated, washed with brine 100 mL and dried over anhydrous magnesium sulfate. The solvent was removed under reduced pressure, and the residue was recrystallized from ethyl acetate 20 mL and hexane 150 mL to give colorless crystals 27.58 g. The crystals were recrystallized from ethyl acetate 50 mL to give benzyl (S)-2-benzyl-3-(cis-hexahydro-2-isoindonyl-carbonyl)propionate 24.45 g as colorless crystals.
Melting point: 107-108°C
¹H-NMR (CDCl₃, 400MHz) δ ppm:
1.3-1.65 (8H, m), 2.1-2.35 (3H, m), 2.55-2.7 (1H, m), 2.8-2.9 (1H, m), 3.0-3.45 (6H, m), 5.0-5.2 (2H, m), 7.1-7.4 (10H, m)
IR(KBr): 1735, 1630cm⁻¹

### Example 2

### Benzyl (S)-2-benzyl-3-(cis-hexahydro-2-isoindonylcarbonyl)-propionate

(S)-2-Benzyl-3-(cis-hexahydro-2-isoindonylcarbonyl)-propionic acid 3.67 g and benzyl alcohol 1.5 g were dissolved in ethyl acetate 20 mL, and N,N'-dicyclohexylcarbodiimide 2.63 g was added to the solution under ice-cooling with stirring. The mixture was stirred for 3 days at room temperature. The insoluble materials were filtered off, and the filtrate was washed with a saturated aqueous sodium bicarbonate solution and dried over anhydrous magnesium sulfate. The solvent was removed under reduced pressure, and the residue was dissolved in a small amount of ethyl acetate. Hexane was added to the solution, and the precipitated crystals were collected by filtration to give benzyl (S)-2-benzyl-3-(cis-hexahydro-2-isoindonylcarbonyl)propionate 3.78 g as colorless crystals. The physical properties were consisted with those of the compound obtained in Example 1.

### Example 3

### Calcium bis[(S)-2-benzyl-3-(cis-hexahydro-2-isoindolinyl-carbonyl)propionate] dihydrate

Benzyl (S)-2-benzyl-3-(cis-hexahydro-2-isoindonyl-carbonyl)propionate 4.06 g was dissolved in methanol 30 mL, and 2N aqueous sodium hydroxide solution 7.5 mL was added to the solution. The mixture was stirred overnight at room temperature. The reaction mixture was concentrated under reduced pressure, and the residue was dissolved in water and washed with ethyl acetate. To the aqueous layer was added 2N hydrochloric acid 7.5 mL, and the mixture was extracted with ethyl acetate. The organic layer was washed with brine and dried over anhydrous magnesium sulfate. The solvent was removed under reduced pressure, and the residue was dissolved in ethanol 40 mL. 2N Aqueous sodium hydroxide solution 5.0 mL was added to the solution, and the mixture was added dropwise to a solution of calcium chloride 2.22 g in water 40 mL with stirring vigorously. Ethanol 10 mL and water 20 mL were added to the reaction mixture and then the precipitated crystals were collected by filtration. The crystals were recrystallized from ethanol and water to give calcium bis [(S)-2-benzyl-3-(cis-hexahydro-2-isoindolinylcarbonyl)-propionate] dihydrate 2.47 g as colorless crystals (the yield from (S)-benzylsuccinic acid was 47%).
Chemical purity: 100%
¹H-NMR(CDCl₃, 400MHz) δ ppm:
1.15-1.5 (16H, m), 1.9-2.4 (6H, m), 2.55-3.1 (14H, m), 3.2-3.5 (6H, m), 7.1-7.3 (10H, m)
IR(KBr): 1660, 1625cm⁻¹

### Example 4

### Calcium bis[(S)-2-benzyl-3-(cis-hexahydro-2-isoindolinyl-carbonyl)propionate] dihydrate

Benzyl (S)-2-benzyl-3-(cis-hexahydro-2-isoindolinyl-carbonyl)propionate 4.05 g was dissolved in methanol 30 mL, and 10% palladium carbon 400 mg was added to the solution. After the mixture was stirred for 2 hours under a hydrogen atmosphere at atmospheric pressure at room temperature, the catalyst was filtered off and then the solvent was removed under reduced pressure. The residue was dissolved in ethanol 80 mL, and the solution to which 2N aqueous sodium hydroxide solution 5.0 mL had been added was added to to dropwise to a solution of calcium chloride 2.22 g in water 50 mL with stirring vigorously. The precipitated crystals were collected by filtration. The crystals were recrystallized from ethanol and water to give calcium bis[(S)-2-benzyl-3-(cis-hexahydro-2-isoindolinyl-carbonyl)propionate] dihydrate 2.73 g as colorless crystals (the yield from (S)-benzylsuccinic acid was 52%). The physical properties were consisted with those of the compound obtained in Example 3.
Chemical purity: 100%

### Comparative Example 1

### Calcium bis[(S)-2-benzyl-3-(cis-hexahydro-2-isoindolinylcarbonyl)propionate] dihydrate

(S)-2-Benzyl-3-(cis-hexahydro-2-isoindolinylcarbonyl)-propionic acid 3.06 g was dissolved in ethanol 20 mL, and the solution to which 2N aqueous sodium hydroxide solution 5.0 mL had been added was added dropwise to a solution of calcium chloride 2.22 g in water 40 mL whilst stirring vigorously. Ethanol 30 mL was added to the reaction mixture. The precipitated crystals were collected by filtration and washed with 10% aqueous ethanol to give colorless crystals 3.06 g (the yield from (S)-benzylsuccinic acid was 87%). The obtained crystals 3.06 g were recrystallized from ethanol and water to give calcium bis[(S)-2-benzyl-3-(cis-hexahydro-2-isoindolinylcarbonyl)propionate] dihydrate 2.09 g as colorless crystals (the yield from (S)-benzylsuccinic acid was 59%).
Chemical purity: 95.5%

The crystals were recrystallized again from ethanol and water to give calcium bis [(S)-2-benzyl-3-(cis-hexahydro-2-isoindolinylcarbonyl)propionate] dihydrate 1.80 g (the yield from (S)-benzylsuccinic acid was 51%). The physical properties were consistent with those of the compound obtained in Example 3.
Chemical purity: 97.4%

## Claims

1. A process for producing a benzylsuccinic acid derivative represented by the formula: 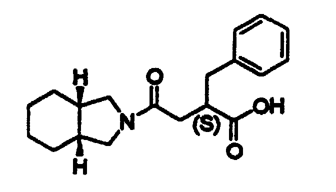 (wherein the carbon atom marked with (S) represents a carbon atom in S configuration) and salts thereof which comprises debenzylating a benzyl ester derivative represented by the formula: 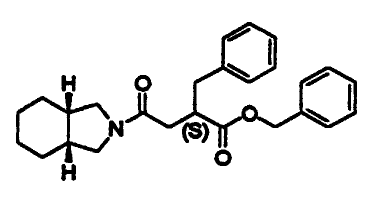 (wherein the carbon atom marked with (S) represents a carbon atom in S configuration) and converting the resulting benzylsuccinic acid derivative into a salt thereof as occasion demands.

2. A process for producing a benzylsuccinic acid derivative represented by the formula: 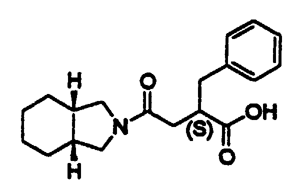 (wherein the carbon atom marked with (S) represents a carbon atom in S configuration) and salts thereof which comprises allowing a benzylsuccinic acid derivative represented by the formula: 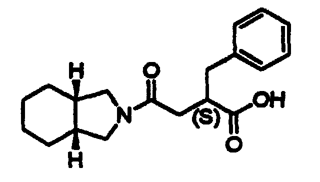 (wherein the carbon atom marked with (S) represents a carbon atom in S configuration) and salts thereof to react with a compound represented by the general formula: 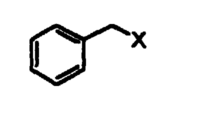 (wherein X represents a hydroxy group, a chlorine atom or a bromine atom) to give a benzyl ester derivative represented by the formula: 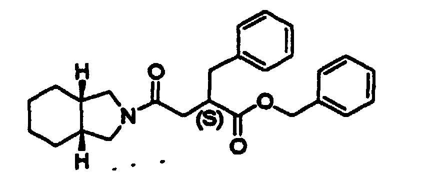 (wherein the carbon atom marked with (S) represents a carbon atom in S configuration), debenzylating the same and converting the resulting benzylsuccinic acid derivative into a salt thereof as occasion demands.

3. A use of a benzyl ester derivative represented by the formula: 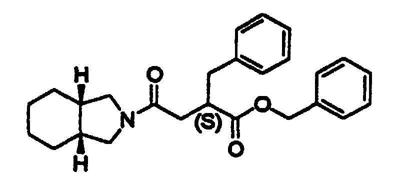 (wherein the carbon atom marked with (S) represents a carbon atom in S configuration) in the production of a benzylsuccinic acid derivative represented by the formula: 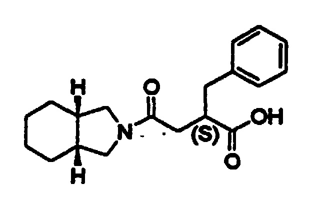 (wherein the carbon atom marked with (S) represents a carbon atom in S configuration) and salts thereof.

4. A benzylsuccinic acid derivative represented by the formula: 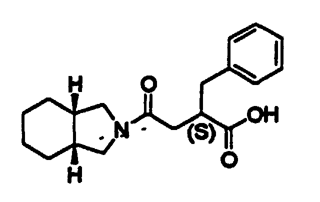 (wherein the carbon atom marked with (S) represents a carbon atom in S configuration) and salts thereof produced by debenzylating a benzyl ester derivative represented by the formula: 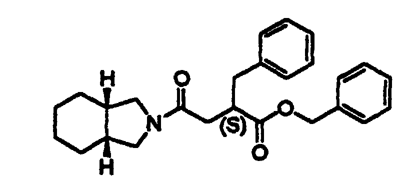 (wherein the carbon atom marked with (S) represents a carbon atom in S configuration) and converting into a salt thereof as occasion demands.
